# EUROPEAN PATENT APPLICATION

(11) **EP 3 243 548 A1**
(43) Date of publication of application: **15.11.2017**
(21) Application number: 16169464.1
(22) Date of filing: 12.05.2016
(51) Int. Cl.: A61M 39/02, A61M 39/10

(54) **PRIMING DEVICE**

(71) Applicant: Renishaw plc, Gloucestershire GL12 8JR (GB)
(72) Inventor: The designation of the inventor has not yet been filed
(74) Representative: Dunn, Paul Edward

(57) **Abstract**

At its most general, the present invention proposes a priming device and method for priming a multi-conduit fluid connector device of a percutaneous fluid access apparatus or subcutaneous fluid access apparatus, in which device and method multiple fluid channels are each arranged to connect to a respective conduit of the multi-conduit fluid connector device to permit deliver of priming liquid from each conduit to a respective fluid channel, and an indicator associated with each respective fluid channel is arranged to indicate the presence of priming fluid within that fluid channel. The priming device of the present invention provides a solution to the problems of known priming methods. The indicators of the multiple fluid channels ensure that it is possible to readily detect when each of the conduits of the multi-conduit fluid connector device has been correctly primed such that any air is expelled. Further, this ready detection ensures that the clinician/user does not have to observe the priming process at a close distance, so reducing the possibility of contamination. Furthermore, it is not necessary for the conduits of the multi-conduit fluid connector device to be exposed during the priming process, so further reducing the contamination risk.

## Description

### FIELD OF THE INVENTION

The present invention relates to a percutaneous or subcutaneous fluid access apparatus, and more specifically to a system for priming a multi-conduit fluid connector device for an implanted percutaneous or subcutaneous fluid access apparatus to deliver therapeutic agents to a subject.

### BACKGROUND OF THE INVENTION

The delivery of therapeutic agents to a particular site in the body, such as the brain, can pose a number of problems. A patient may have to undergo repeated surgery to access a site to which a therapeutic agent is to be delivered. A percutaneous or transcutaneous access device is a device which crosses the skin, providing a connection between the interior and exterior of the body via which a therapeutic agent may be administered. Such devices may include a part thatis implanted below the skin to allow permanent access to a specific site, for example via a catheter in the brain. Subcutaneous access devices that are fully buried under the skin and accessed by passing needles or similar through the skin are also known.

The device described in WO 2013/117659 comprises a percutaneous access apparatus that can be anchored directly to the skull of a patient. The implanted device comprises one or more ports that provide access to a specific site of the brain via one or more catheters. The therapeutic agent is delivered through an external multi-needle fluid connector that attaches to the implanted device. Each needle delivers the therapeutic agent to a respective one of the ports of the implanted device to deliver the therapeutic agent from the multi-needle connector to the subject.

The multi-needle fluid connector of WO2013/117659, and other similar devices, must be primed before attachment to the implanted device in order to ensure that there is no air present in any of the supply channels carrying the therapeutic agent, i.e. the needles and supply lines. The priming fluid used for such a priming operation may comprise an inert fluid such as saline or artificial cerebral spinal fluid (CSF), or may comprise a disposable quantity of the therapeutic agent. If air is present in any of the supply channels before connection to the port, that air can block the fluid distribution from the connector to the port and/or from the catheter to the patient. The presence of air may result in administering the wrong dose of the therapeutic agent or even alter the distribution of the therapeutic agent inside the body (e.g. brain) of the subject.

The present invention proposes a device and method to aid the process of priming a multi-conduit connector of a percutaneous or subcutaneous access apparatus.

### SUMMARY OF THE INVENTION

At its most general, the present invention proposes a priming device and method for priming a multi-conduit fluid connector device of a percutaneous fluid access apparatus or subcutaneous fluid access apparatus, in which device and method multiple fluid channels are each arranged to connect to a respective conduit of the multi-conduit fluid connector device to permit delivery of priming liquid from each conduit to a respective fluid channel, and an indicator associated with each respective fluid channel is arranged to indicate the presence of priming fluid within that fluid channel.

As discussed above, it is essential to prime the multi-conduit fluid connector device of a percutaneous or subcutaneous access device before the two devices are interconnected, in order to ensure that there is no air present in any of the supply channels carrying the therapeutic agent when the therapeutic agent is administered.

The inventors have found that during priming a user must expose the conduits of the multi-conduit fluid connector device in order to observe fluid flow from each conduit of the multi-conduit connector device to verify priming was successful. Such observation must necessarily be carried out at a close distance, which increases the risk of contamination of the multi-conduit fluid connector device. Moreover, the conduits, e.g. needles, are usually very small and arranged close together; it is therefore often difficult to establish which conduit an observed fluid flow has originated from, leading to the potential for a user to erroneously believe that a conduit has been correctly primed.

Prior art percutaneous access devices may include air filters to lessen the potential effects of incorrect or incomplete priming of the connector device, but avoiding the need to include such air filters can be advantageous.

A first aspect of the present invention provides a system for priming a multi-conduit fluid connector device of a percutaneous fluid access apparatus or subcutaneous fluid access apparatus, comprising: a multi-conduit fluid connector device comprising a plurality of fluid conduits arranged to receive a priming liquid; and a priming device comprising multiple fluid channels each for connection to a respective conduit of the multi-conduit fluid connector device to permit delivery of priming liquid from each conduit to a respective fluid channel, and an indicator associated with each respective fluid channel for indicating the presence of priming liquid within the fluid channel.

The priming device of the present invention provides a solution to the problems discussed above in relation to prior art priming methods. The indicators of the multiple fluid channels ensure that it is possible to readily and reliably detect when each of the conduits of the multi-conduit fluid connector device has been correctly primed such that any air is expelled. Further, this ready detection ensures that the clinician/user does not have to observe the priming process at a close distance, so reducing the possibility of contamination. Furthermore, it is not necessary for the conduits of the multi-conduit fluid connector device to be exposed during the priming process, so further reducing the contamination risk.

In embodiments in which the priming device is supplied by the manufacturer or supplier pre-assembled with the multi-conduit connector device, a further advantage of the present invention is that the fluid conduits of the multi-conduit fluid connector device are shielded from damage or contamination during storage or transport by the priming device. Such damage during storage or transportation may have detrimental effects when the user attempts to connect the multi-conduit fluid connector device to percutaneous or subcutaneous fluid access apparatus. If, for example, the conduits of the multi-conduit fluid connector device comprise needles that are buckled or distorted, the user may put unnecessary force on the percutaneous or subcutaneous fluid access apparatus during connection, risking damage to the percutaneous or subcutaneous fluid access apparatus. This arrangement can also prevent needle-stick injuries to those handling the priming device and connector device assembly during transport or storage.

The system of the present invention also reduces the risk of needle-stick injury for the user/clinician during priming, since the user does not come in contact with the fluid conduits of multi-conduit connector device which may comprise sharp edges such as needle points.

In embodiments in which the therapeutic agent is used as the priming fluid, the system of the present invention also reduces the risk of the user coming in contact with the therapeutic agent. The priming device may be configured to retain any excess priming fluid after detachment from the multi-conduit fluid connector device (e.g. it may be self-sealing). This is particularly important in applications in which it may be unsafe for the therapeutic agent to contact a person's skin.

Each fluid channel of the priming device preferably has an open distal end to permit venting of air. In some embodiments each fluid channel may comprise a membrane or substrate such as a hydrophobic or hydrophilic membrane which enables air to escape from the distal end of the channel, but prevents liquid from escaping.

In some embodiments the indicators each comprise a visual indicator to provide a visual indication of the presence of priming fluid within the fluid channel. A visual indication enables a quick and easy way for a user/clinician to determine whether priming has been successful. Moreover, a visual indication enables the clinician to visually observe the status of priming of all the fluid channels simultaneously.

In other embodiments the indicators may comprise an audible indicator such as a buzzer or other audible signal.

Each visual indicator may comprise a length of substantially transparent hollow tube defining the respective fluid channel. An open end of each conduit of the connector device may be inserted into or up to an open end of each respective hollow tube during priming.

This arrangement provides a visible reservoir for receiving the priming liquid from the respective fluid conduit of the connector device. The user/clinician can determine whether priming has been successful for each of the conduits of the connector device by observing whether the priming liquid is present in each of the hollow tubes. Moreover, the reservoir of priming liquid in each tube surrounds the exit opening of its respective conduit, thus ensuring that any back-track of priming liquid caused by pressure differentials experienced during movement of the connector device results in a back-track of priming liquid from the reservoir and not air. The tubes are each sufficiently rigid and/or long to maintain the reservoir of fluid during movement of the connector device.

Another advantage of the hollow tube arrangement is that it is inexpensive to implement. The priming device may therefore be disposable.

Each hollow tube preferably has a proximal end for directly attaching to the respective fluid conduit (e.g. needle). For fluid conduits comprising needles, each tube may be separately attachable to the respective needle, or alternatively the respective tubes may be collectively attachable to the plurality of needles. In some embodiments the tubes are formed as a multi-lumen tube.

Each hollow tube preferably has an open distal end opposite the needle. The distal end may alternatively be supplied to the user in a closed state, and subsequently opened by e.g. cutting with a scalpel, before use. In some embodiments each hollow tube may comprise a membrane or substrate such as a hydrophobic or hydrophilic membrane which enables air to escape from the distal end of the hollow tube, but prevents liquid from escaping.

The hollow tubes are preferably retained by a connecting member which serves to both provide the connection between the priming device and the connector device, and to correctly locate/align the proximal ends of the tubes with respect to the conduits of the connector device. In some embodiments the connecting member permits connection in only one orientation to ensure that each tube is associated with only one conduit. The tubes and/or connecting member may be labelled or colour-coded to indicate which conduit each hollow tube is associated with.

The multi-conduit fluid connector device preferably comprises an attachment member configured for attachment of the connector device to a percutaneous fluid access device and for attachment of the connector device to the priming device. In this way, the connector device has only one means for attaching both the priming device and the percutaneous fluid access device, thus reducing complexity of this device. Additionally, this feature facilitates an easy process of removing the priming device from the connector device and subsequently connecting the connector device to the percutaneous or subcutaneous access device. It also enables a user to become familiarised with the attachment member and attachment process before connecting the connector device to the implanted access device.

The attachment member is preferably arranged to orientate the priming device relative to the multi-conduit fluid connector device to ensure alignment between each conduit and its respective fluid channel. In this way, correct alignment can always be assured. The attachment member may have one or more geometrical features, such as, for example, protrusions, grooves, recesses or indentations, to achieve correct orientation. The connector device may have corresponding geometrical features to enable a keyed connection.

In some embodiments the fluid conduits of the multi-conduit fluid connector device each comprise a hollow needle. The hollow needles may each have a sharp point arranged to pierce a septum, or other membrane, extending across an opening to a fluid port of the subcutaneous or percutaneous fluid access device. Until the septum is pierced, fluid cannot flow from the needle into the port. In other embodiments, the fluid conduits may each comprise a micro-tube or other tube-like conduit comprising a valve which is normally closed but can be opened upon connection of the connector device to the percutaneous or subcutaneous access device.

In embodiments in which the conduits comprise needles, the multi-conduit fluid connector device preferably has a connection configuration in which the needles do not protrude from the multi-conduit connector device and a connected configuration in which the needles protrude into the fluid channels of the priming device, and the priming device can be connected to or disconnected from the multi-conduit connector device only in the connection configuration.

Such an arrangement has several advantages. Firstly, it ensures that the needles are protected from damage or contamination during transport and storage. Secondly, it protects the user from needle-stick injuries during connection and disconnection of the priming device to/from the connector device. Thirdly, it ensures that the needles are not bent or otherwise damaged during connection and disconnection of the priming device to/from the connector device. Fourthly, it ensures that the needles do not damage the septum when establishing a fluid connection with a percutaneous fluid access device.

In some embodiments each indicator includes an electronic liquid sensor coupled to an output device. Thus, the sensor is able to detect when liquid is present in the fluid channel, and therefore can detect when priming has been successful for that fluid channel. For example, the output device could include a light source, such as an LED, to visually indicate successful priming. Alternatively, the output device could provide an audible signal, or could comprise a user device such as a mobile phone, tablet or laptop which displays the priming status via a user interface, based on an output signal from the sensor.

In alternative embodiments the indicator includes a substrate which is adapted to undergo a visually observable change on contact with liquid. Thus, the user can visually observe that priming has been successful.

Each conduit of the multi-conduit connector device and/or each fluid channel of the priming device may include a non-return valve. In this way, priming liquid is prevented from back-tracking through the conduits in the event that the connector device is moved sufficiently before attachment to the access device to cause a pressure differential.

The system may comprise a liquid source configured to supply priming fluid through the conduits and into the fluid channels to expel air from the conduits, and optionally from at least a portion of the fluid channels. The liquid source may comprise a pump, for example a known syringe pump of the type typically available on hospital wards.

In preferred embodiments the multi-conduit fluid connector device is configured for attachment to a percutaneous fluid access device or subcutaneous fluid access device comprising an extracorporeal portion, and a plurality of ports accessible from the extracorporeal portion. The extracorporeal portion preferably comprises an attachment member corresponding to the attachment member of the connector device, to achieve connection between the connector device and the access device. The plurality of ports are preferably configured to be in fluid communication with a catheter or other tube implanted in the subject to deliver a therapeutic agent to that subject. Each conduit of the connector device is preferably associated with a respective one of the ports of the access device.

Another aspect of the invention provides an apparatus comprising a system according to the first aspect, and a percutaneous or subcutaneous access apparatus configured for attachment to the multi-needle fluid connector device, the percutaneous or subcutaneous access apparatus comprising an extracorporeal portion and a plurality of ports accessible from the extracorporeal portion, each port being for connection to a respective one of the plurality of needles of the multi-needle fluid connector device to permit delivery of a fluid from each needle to a respective port. Thus, the percutaneous or subcutaneous access apparatus may be attached to the multi-needle fluid connector device in a fluid delivery mode, and the priming device may be attached to the multi-needle fluid connector device in a priming mode.

In preferred embodiments the multi-conduit fluid connector device comprises an attachment member configured for attachment to the percutaneous or subcutaneous fluid access device in a fluid delivery mode, and for attachment to the priming device in a priming mode. Thus, the existing attachment member that is used for attaching the multi-needle connector device to the percutaneous or subcutaneous fluid access device can be used for attaching the priming device to the connector device. This reduces overall design complexity and ensures that the system is easy to use.

The attachment member is preferably arranged to orientate the priming device relative to the multi-conduit fluid connector device to ensure alignment between each conduit and its respective fluid channel. In this way, correct alignment can always be assured. The attachment member may have one or more geometrical features, such as, for example, protrusions, grooves, recesses or indentations, to achieve correct orientation. The connector device may have corresponding geometrical features to enable a keyed connection.

The multi-conduit fluid connector device may comprise more than two fluid conduits. The priming device may comprise more than two fluid channels. The multi-conduit fluid connector device may comprise four or more fluid conduits. The priming device may comprise four or more fluid channels.

A second aspect of the invention provides a method of priming a multi-conduit fluid connector device of a percutaneous fluid access apparatus or subcutaneous fluid access apparatus, the multi-conduit fluid connector device comprising a plurality of fluid conduits, the method comprising: connecting the multi-conduit fluid connector device to a liquid source; providing a priming device connected to the multi-conduit fluid connector device, the priming device comprising multiple fluid channels such that each fluid channel is connected to a respective conduit of the multi-conduit fluid connector device, and having an indicator associated with each respective fluid channel; and supplying priming liquid from the liquid source through the conduits and into the fluid channels to expel air from the conduits until each indicator indicates the presence of priming liquid within each of the fluid channels.

The advantages of the method according to the second aspect are as set out above in relation to the first aspect. Additionally, the optional and/or desirable features described above in relation to the first aspect are equally applicable to the second aspect, either individually or in any combination.

In preferred embodiments the fluid conduits of the multi-conduit fluid connector device each comprise a hollow needle, and the method further comprises providing the multi-conduit fluid connector device in a connecting configuration in which the hollow needles do not protrude from the multi-conduit fluid connector device, and, only after the multi-conduit fluid connector device is connected to the priming device, deploying the hollow needles to a connected configuration in which they protrude into the fluid channels of the priming device.

Such an arrangement has several advantages. Firstly, it ensures that the needles are protected from damage or contamination during transport and storage. Secondly, it protects the user from needle-stick injuries during connection and disconnection of the priming device to/from the connector device. Thirdly, it ensures that the needles are not bent or otherwise damaged during connection and disconnection of the priming device to/from the connector device.

The method may further comprise, after the presence of priming liquid within each of the channels has been indicated: disconnecting the priming device from the multi-conduit fluid connector device; and attaching the multi-conduit fluid connector device to a percutaneous fluid access device or subcutaneous fluid access device comprising an extracorporeal portion and a plurality of ports accessible from the extracorporeal portion, such that the conduits provide fluid communication between the conduits and respective ones of the ports.

In such methods the step of connecting the multi-conduit fluid connector device to the priming device may include connecting the priming device to an attachment member of the multi-conduit fluid connector device, and the step of attaching the multi-conduit fluid connector device to a percutaneous fluid access device may include connecting the percutaneous fluid access device to the attachment member.

In this way, the connector device needs to have only one means for attaching both the priming device and the percutaneous fluid access device, thus reducing complexity of this part. Additionally, this feature facilitates an easy process of removing the priming device from the connector device and subsequently connecting the connector device to the percutaneous or subcutaneous access device. It also enables a user to become familiarised with the attachment member and attachment process before connecting the connector device to the access device.

Similarly, in some embodiments the method further comprises attaching the multi-needle connector device to an attachment mechanism for attaching the multi-needle fluid connector device to an extracorporeal portion of a percutaneous fluid access device prior to connecting the multi-needle connector device to the liquid source and the priming device. In this way, the connector device is placed in a condition in which it is ready for attachment to the percutaneous fluid access device prior to the priming operation.

A further aspect of the invention provides a priming device for priming a multi-conduit fluid connector device of a percutaneous fluid access apparatus or subcutaneous fluid access apparatus, the multi-conduit fluid connector device comprising a plurality of fluid conduits arranged to receive a priming liquid, the priming device comprising multiple fluid channels each for connection to a respective conduit of the multi-conduit fluid connector device to permit delivery of priming liquid from each conduit to a respective fluid channel, and an indicator associated with each respective fluid channel for indicating the presence of priming liquid within the fluid channel.

Each of the optional and/or desirable features described above in relation to the other aspects of the invention are also applicable to the above further aspect of the invention, either individually or in any combination thereof.

Also described herein is a system for priming a fluid connector device (e.g. of a percutaneous fluid access apparatus or subcutaneous fluid access apparatus) comprising: a fluid connector device comprising one or more fluid conduits arranged to receive a liquid (e.g. a priming liquid); and a priming device comprising one or more fluid channels each for connection to a respective conduit of the fluid connector device to permit delivery of liquid from each conduit to a respective fluid channel. The priming device may also comprise an indicator associated with each respective fluid channel for indicating the presence of liquid (e.g. priming liquid) within the fluid channel. The fluid connector device may be a multi-conduit fluid connector device comprising a plurality of fluid conduits. The system may include any of the other features, alone or in combination, that are described above for the various aspects of the invention.

### BRIEF DESCRIPTION OF THE DRAWINGS

Embodiments of the invention will now be described with reference to the accompanying drawings, in which:
Figure 1 schematically illustrates a drug delivery system for which the priming device and method of the present invention may be used;
Figures 2A and 2B show an isometric view and a section view, respectively, of the percutaneous port (also referred to as a percutaneous access device) of the drug delivery system of Figure 1;
Figure 3 shows an isometric view of a connector device suitable for connecting to the percutaneous port of Figures 2A and 2B;
Figures 4A and 4B show an isometric view and an exploded view, respectively, of the connector device of Figure 3 connected to a priming device according to an embodiment of the invention;
Figures 5A, 5B and 5C show isometric (5A and 5B) and exploded (5C) views of the priming device of Figures 4A and 4B; and
Figures 6A, 6B and 6C show section views of the connector device of Figure 3 in a retracted configuration (Figure 6A), the connector device and priming device assembly of Figure 4a in a retracted configuration (Figure 6B), and the connector device and priming device assembly in a deployed configuration (Figure 6C).

### DETAILED DESCRIPTION OF EMBODIMENT(S)

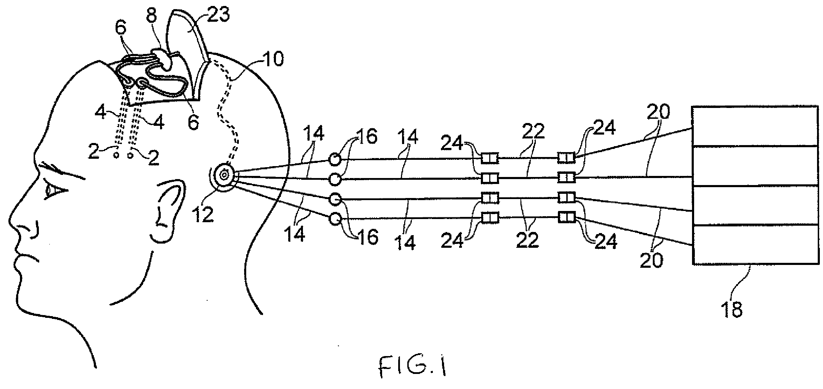

Referring to Figure 1, an overview of an apparatus for delivering fluid to the brain is illustrated when implanted in a subject. The priming device and method according to the present invention are suitable for use with the percutaneous access apparatus 12 of the illustrated fluid delivery apparatus. The priming device and method according to the invention are also suitable for use with other percutaneous and subcutaneous access devices, as discussed further below.

The apparatus comprises four fine catheters 2, each catheter being inserted into the brain via a previously implanted guide tube 4 (although it should be noted that only two of these are shown in Figure 1). Suitable stereotactic insertion apparatus and methods have been described elsewhere previously, for example see US7329262 for details of a stereoguide based catheter insertion procedure. Supply tubing 6 runs from each catheter 2 to a hub 8. The hub 8 is connected by a length of multi-lumen tubing 10 to percutaneous access apparatus 12. The catheters 2, guide tubes 4, supply tubing 6, hub 8 and multi-lumen tubing 10 are all subcutaneously implantable (i.e. buried beneath the skin of the patient).

The percutaneous access apparatus 12 comprises a percutaneous fluid access device that is anchored directly to the skull of the patient. The percutaneous fluid access device comprises an extracorporeal portion to which an associated connector device is releasably attached. The percutaneous access apparatus 12 thus enables a fluidic link to the implanted catheters 2 to be established when required. In particular, the arrangement provides a separate, isolated, fluidic pathway to each catheter 2. More details about the percutaneous access apparatus 12 are provided below.

Outside of the body, the connector device (described below) of the percutaneous access apparatus 12 is linked to four external supply tubes 14. Each supply tube 14 includes an in-line bacterial and/or air filter 16. A four channel syringe pump 18 (which may comprise four separate single channel syringe pumps) is also provided. An outlet tube 20 from each channel of the syringe pump 18 is linked to one of the external supply tubes 14 via a drug storage tube 22. As will be explained in more detail below, each drug storage tube 22 is preloaded with a desired volume of therapeutic agent allowing the syringe pump 18 to be loaded with an inert solution (e.g. saline or artificial CSF). Fluidic connections between the drug storage tube 22 and the outlet tubes 20 and supply tubes 14 are made using low dead volume Luer lock connectors 24 of the type described in more detail below.

In use, the catheters 2, guide tubes 4, supply tubing 6, hub 8 and multi-lumen tubing 10 are all subcutaneously implanted in the subject (i.e. the skin flap 23 showed in a raised position in Figure 1 is folded down and sutured in place). The percutaneous fluid access device 100 of the percutaneous access apparatus 12 is also secured in place (e.g. attached to the skull and left protruding through the scalp) thereby providing the required fluid connection as and when required. These components are preferably suitable for long term implantation within a subject. For example, they may be designed to remain implanted for months or years.

When delivery of therapeutic agent is required, the connector device 130 (described below) is attached to the percutaneous fluid access device 100 of the apparatus 12. The supply tubes 14 (pre-primed with inert fluid in accordance with the present invention, as discussed below) are then connected to the syringe pump via drug storage tubes 22 that contain the required dosage of therapeutic agent that is to be delivered. Each channel of the syringe pump is arranged to expel inert fluid (saline, artificial CSF etc.) thereby pushing the therapeutic agent through the apparatus and expelling it from the tips of each catheter 2. The rate of fluid flow can be precisely controlled using the syringe pump 18 and the amount of therapeutic agent can be precisely set by defining the volume of the drug storage tubes 22. It is possible for fluid delivery to be continuous or intermittent. Fluid may also be delivered through all, or just some, of the catheters in parallel and/or it may be delivered sequentially through a sub-set of one or more catheters in turn. The precise delivery protocol can be set by a clinician.

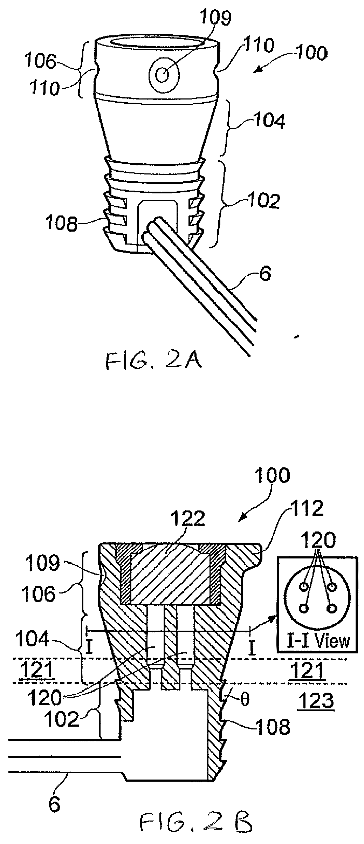

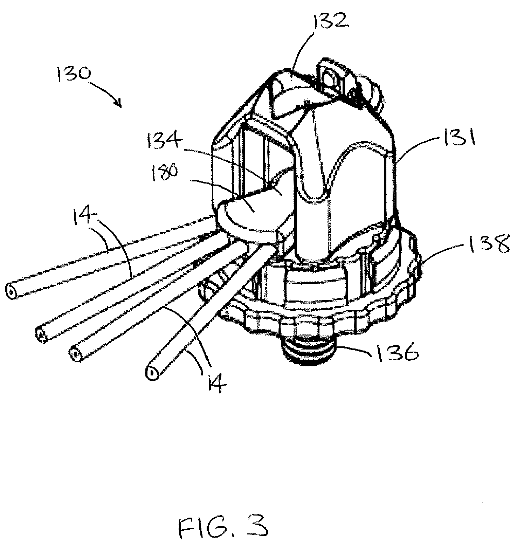

Figures 2A and 2B illustrate the percutaneous fluid access device 100 that is implanted in the subject and Figure 3 shows the external connector device 130 that attaches to the percutaneous fluid access device 100 whenever fluid delivery is required.

Referring to Figures 2A and 2B, the percutaneous fluid access device 100 comprises a subcutaneous portion 102, a percutaneous portion 104 and an extracorporeal portion 106.

The subcutaneous portion 102 is substantially cylindrical with protruding ribs 108 that enable secure attachment of the device to a hole formed in the skull via an interference or press fit. The external surface of the subcutaneous portion 102 is also roughened to promote osseointegration after implantation. The ribs 108 have an inclined surface that is at an angle θ of between 15 and 35 degrees to the longitudinal axis; this helps retain the device securely in place after implantation.

The percutaneous portion 104 (which can also be termed a transcutaneous portion) is the part of the device that passes through the skin. The surface of the percutaneous portion 104 is also roughened to promote skin in-growth after implantation thereby reducing the risk of infection. The percutaneous portion 104 is conical (i.e. it increases in diameter from skin surface) with an angle from the vertical of between 5 and 40 degrees.

The extracorporeal portion 106 is the part of the device that protrudes above the outer surface of the dermis. The extracorporeal portion 106 thus has a smooth surface to prevent tissue in-growth; such a smooth surface also allows it to be easily cleaned thereby reducing the chance of bacterial retention.

The extracorporeal portion 106 has a substantially cylindrical outer surface with a conical recess 109 and two v-shaped grooves 110 spaced apart around its circumference. A macro-alignment feature 112 is also provided. The conical recess 109 and grooves 110 act as very precise (kinematic) location features for the associated connector device, whilst the macro-alignment feature 112 ensures the connector device is in the approximately correctly orientation prior to attachment. Further details of the connector device 130 are provided below.

As shown in the cross-sectional views of Figure 2B, the percutaneous fluid access device 100 comprises four ports 120. Each port 120 is in fluid communication with a lumen of the multi-lumen supply tube 6. The supply tube 6 exits the subcutaneous portion 102 from its side and, when implanted, runs a short distance in a channel formed in the bone. The four ports 120 are accessible from the extracorporeal portion via a septum 122. In particular, each port 120 comprises an elongate channel having an axis substantially parallel to the longitudinal axis of the device. A single septum 122 that is accessible from the extracorporeal portion seals the end of the channel of all four ports. During fluid delivery, hollow needles of the connector device 130 pierce the septum, enter the channels and thereby provide the required fluid communication with each port. In the absence of an attached connector device, the septum seal provides a fluid seal for all ports that prevents leakage of fluid or ingress of unwanted material (e.g. bacteria etc). Figure 2B also shows in dashed outline the location of the dermal layer 121 and underlying bone 123 when the device is implanted.

Figure 3 illustrates an appropriate connector device 130 for attachment to the percutaneous fluid access device 100. The connector device 130 comprises a connector base 131 having a female attachment socket 132 for securing the connector device 130 to the percutaneous fluid access device 100 in a precisely defined relative position. The connector device 130 also includes a needle holder 134 attached to the end of a shaft 136. The shaft 136 has an external thread that engages a corresponding internal thread of a knurled portion 138. The needle holder 134 is located within a guide channel inside the connector base 131 and rotation of the knurled portion 138 relative to the connector base 131 drives the needle holder 134 back and forth along the channel. After the connector base 131 has been attached to the percutaneous fluid access device 100 via the attachment socket 132, the knurled portion 138 can be rotated to drive the hollow needles held by the needle holder 134 through the septum of the percutaneous fluid access device 100, thereby establishing the required fluid communication. The supply tubes 14 connected to the needles of the needle holder 134 are also shown.

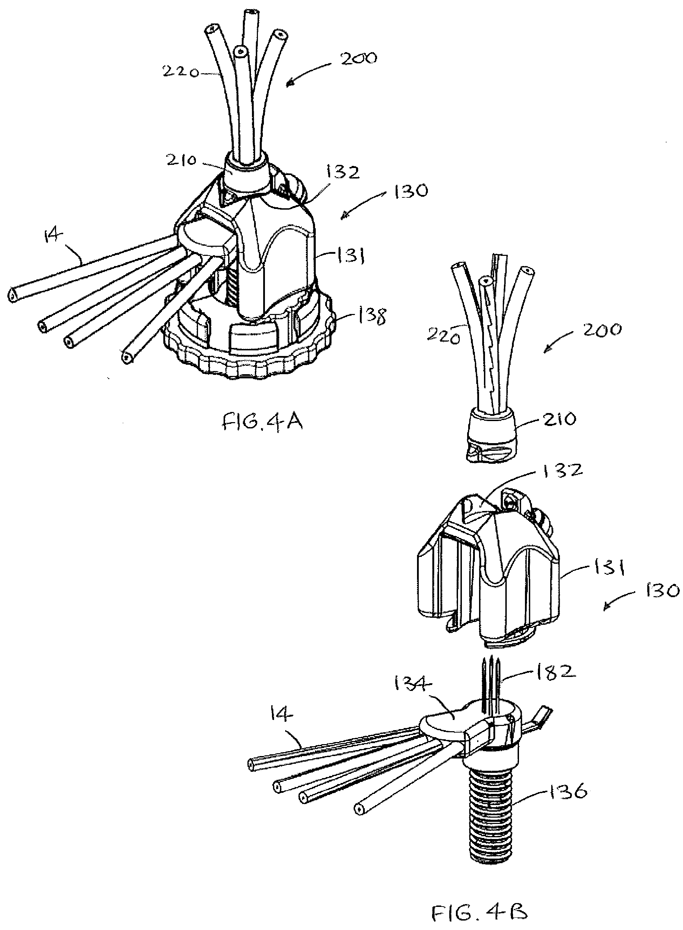

The needle holder 134 comprises a substantially flat, keyhole shaped, supporting member 180. Four hollow needles 182 (see Figure 4b) project perpendicularly from the flat surface of the supporting member. The four hollow needles 182 are spaced apart in a configuration that matches the arrangement of the ports of the percutaneous fluid access device 100. The needle holder 134 also includes four internal channels that provide separate fluidic channels between the lumens of the four hollow needles 182 and the four supply tubes 14. The screw threaded shaft 136 attached to the needle holder 134 is held by the threaded inner surface of the knurled portion 138.

The attachment socket 132 of the connector device 130 permits connection of the connector device 130 to the percutaneous fluid access device 100. The female attachment socket 132 comprises a recess or concave feature which has a corresponding shape to the male extracorporeal portion 106 of the percutaneous fluid access device 100. Thus, the extracorporeal portion 106 comprises a plug part which is arranged to be inserted into the attachment socket 132 via a push fit to achieve attachment of the connector device 130 to the percutaneous fluid access device 100. A locking screw may also be provided to more securely attach the connector device 130 to the percutaneous fluid access device 100.

There is a close fit between the two interfacing parts to ensure that the extracorporeal portion 106 is retained within the attachment port 132 until removed by a user; as mentioned above, a locking screw may also be provided to ensure the parts remain engaged. The macro-alignment feature 112 of the extracorporeal portion 106 comprises a protrusion which extends radially outwardly from the substantially cylindrical portion and engages a correspondingly shaped recess in the attachment port 132 to ensure correct angular alignment between the extracorporeal portion and the attachment port. The conical recess 109 and grooves 110 act as very precise (kinematic) location features for the attachment socket, whilst the macro-alignment feature 112 ensures the connector device is in the correct orientation prior to attachment.

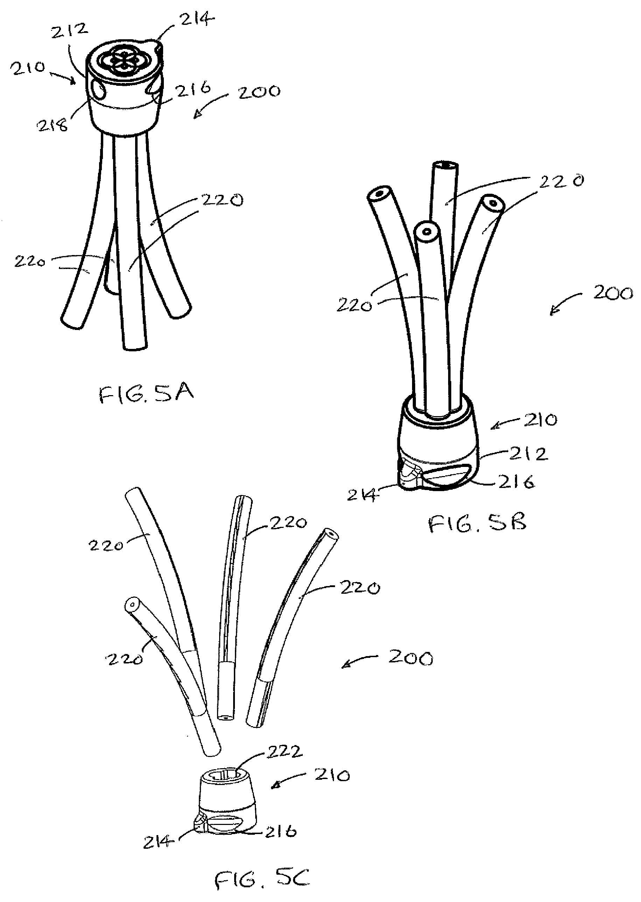

The attachment socket 132 also permits connection of the connector device 130 to a priming device 200. The priming device 200 is shown connected to the connector device 130 in Figure 4A, and Figure 4B shows an exploded view of the assembly of Figure 4A with the knurled portion 138 omitted for clarity. Figures 5A and 5B show isometric views of the priming device itself, while Figure 5C shows an exploded view of the priming device 200.

The priming device 200 is connected to the connector device 130 to permit a priming fluid to be pumped through the supply tubes 14 into the connector device 130 to ensure that there is no air in the fluid path that includes the supply tubes 14 and the needles 182. Thus, the priming device 200 is used to prime the supply tubes 14 and connector device 130 before connection of the connector device 130 to the percutaneous access device 100. The priming fluid may be an inert fluid such as saline, artificial CSF etc., or may be a disposable volume of the therapeutic fluid to be administered to the patient via the percutaneous access device 100.

The priming device 200 comprises a plug portion 210 which retains an openended proximal end of each of four priming tubes 220. The priming tubes 220 each have a distal end which is open to the environment to allow escape of air that has been displaced from the supply tubes 14 and/or needles 182 by the priming process. In some embodiments the priming tubes 220 may each include a hydrophilic or hydrophobic membrane to permit venting of air but prevent priming liquid from being expelled from the distal ends of the priming tubes 220.

In still further embodiments the distal ends of the priming tubes 220 may be initially closed, but cut by the end user (clinician) to open them before use of the priming device. This process may, for example, prevent contamination of the needles 182 in embodiments in which the priming device 200 is supplied to the clinician already attached to the connector device 130.

The plug portion 210 of the priming device 200 is shaped and sized to be inserted into the attachment socket 132 of the connector device 130 to achieve a push fit connection between the priming device 200 and the connector device 130. There is a close fit between the attachment socket 132 and the plug portion 210 to ensure that the connection is maintained until a user disconnects the priming device 200. The plug portion 210 can only be inserted into the attachment socket 132 when the connector device 130 is in the connecting configuration, as described below.

The plug portion 210 has a generally cylindrical body portion 212 from which a locating protrusion 214 projects radially. The locating protrusion 214 corresponds to the correspondingly shaped recess in the attachment socket 132 to ensure correct angular alignment between the plug portion 210 and the attachment socket. The plug portion 210 also has two opposing v-shaped grooves 216 formed in the surface of the cylindrical portion 212, and a conical recess 218 located at an opposing face of the cylindrical portion to the locating protrusion 214. The grooves 216 and conical recess 218 interact with corresponding locating features in the attachment socket 132 of the connector device 130 to act as very precise (kinematic) location features for the attachment socket, whilst the macro-alignment feature 112 ensures the connector device is in the correct orientation prior to attachment.

The priming tubes 220 are retained by a quatrefoil shaped bore 222 which extends through the full axial extent of the plug portion 210, with the proximal end of each tube being retained within a respective one of the four limbs, or lugs, of the bore 222. The bore 222 acts as a locator to position each of the priming tubes 220 with respect to one another and with respect to the plug portion 210. In this way, the open end of each proximal end of each tube is aligned with a respective one of the four needles 182 of the connector device 130 such that, when the priming device 200 is attached to the connector device 130 by insertion of the plug portion 210 into the attachment socket 132, and the needles are in the deployed configuration (see below), the pointed end of each needle extends through the open end of its respective tube 220 so that a tip portion of the needle projects into the tube.

The presence of the locating protrusion 214 and associated locating features 216, 218 of the plug portion 210 ensures that each priming tube 220 is always associated with a particular one of the needles 182 and therefore a particular one of the supply tubes 14. In some embodiments the priming tubes 220 may be each uniquely labelled to identify which supply tube it is associated with, for example by colour coding or with other identifying marks.

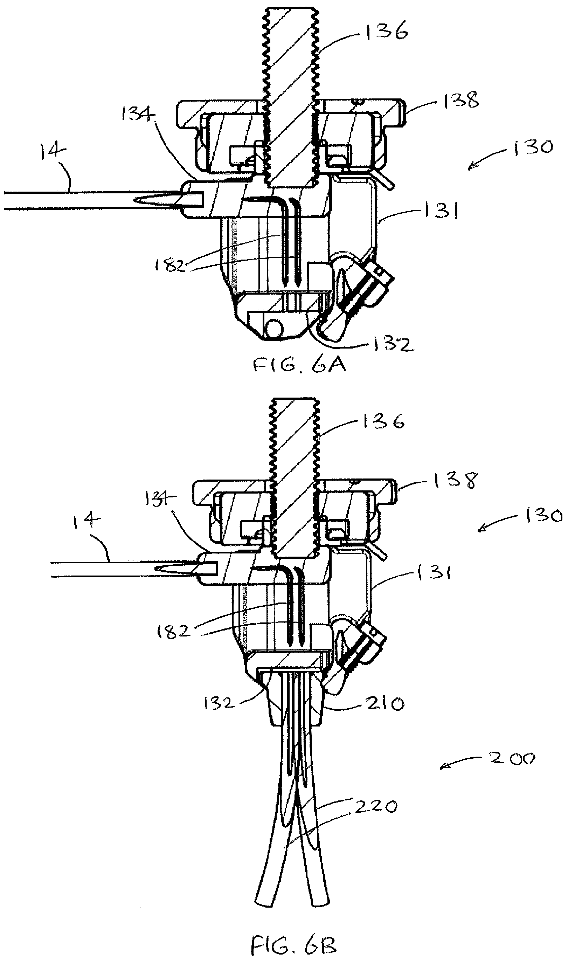

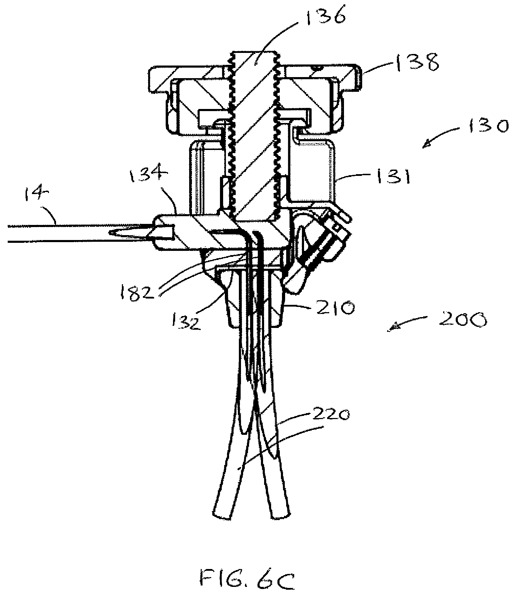

As discussed above, the needle holder 134 of the connector device 130 is located within a guide channel inside the connector base 131, and rotation of the knurled portion 138 relative to the connector base 131 drives the needle holder 134 back and forth along the channel to move the needles 182 between a retracted configuration (Figures 6A and 6B) and a deployed configuration (Figure 6C).

Thus, as shown in Figure 6A, the connector device 130 has a retracted configuration in which the needles 182 are retracted so that they do not protrude from the connector device 130. In the retracted configuration the priming device 200 can be connected to, or disconnected from, the connector device 130 by insertion of the plug portion 210 into the attachment socket 132, as shown in Figure 6B. Before use of the priming device 200, the connector device 130 must be switched to the deployed configuration (Figure 6C) by rotating the knurled portion 138 relative to the connector base 131 in order to deploy the needles 182 so that they protrude from the connector device 130 (extending into the recess provided by the attachment socket 132) and are each inserted into a respective one of the priming tubes 220.

In other embodiments the needles 182 of the connector device 130 may be fixed in position relative to the attachment socket 132. In such embodiments, the connector device 130 has no configurations in which connection between the plug portion 210 and the attachment socket 132 is prevented.

In use, the connector device 130 is arranged in the retracted configuration, and the priming device 200 is then connected to the connector device 130 by insertion of the plug portion 210 into the attachment socket 132. The connector device 130 is then switched to the deployed configuration by turning of the knurled portion 138 so that the needles 182 each extend into a respective one of the priming tubes 220. Priming fluid is then pumped through the supply tubes 14 into the connector device 130, optionally by the pump 18.

The priming fluid travels from each of the supply tubes 14 into each respective needle 182, and from each needle 182 into each respective priming tube 220. This priming process serves to evacuate air pockets from the supply tubes 14 and needles 182. Thus, the presence of priming fluid within each priming tube 220 indicates that the respective needle 182 and supply tube 14 have been successfully primed and do not retain any air. Only when all four priming tubes 220 are observed to contain priming fluid is the connector device 130 successfully primed.

After the priming process is completed the priming device 200 is disconnected from the multi-needle connector device 130 and the connector device 130 is subsequently attached to the percutaneous access device 100 for delivery of the therapeutic agent to the patient. During this process the connector device may remain connected to, or be disconnected from, the pump 18, and the pump 18 may be stopped or may be operational.

It is desirable to prevent the priming fluid from being able to track back through the priming tubes 220 such that air can re-enter the needles 182 after the priming operation has been completed and before the connector device 130 is attached to the implanted percutaneous access device 100. Such a back flow may occur when the connector device 130 and priming device 200 assembly is raised and lowered before attachment to the patient, for example, when pressure differentials may occur. It is therefore desirable to either prevent back-tracking of fluid and/or ensure that any such back-tracking does not enable air to enter the needles 182.

Entry of air into the needles caused by back-tracking of priming fluid may be prevented by ensuring that the priming tubes 220 are sufficiently rigid to prevent bending of the tubes which may lead to air entry, and/or by ensuring that the priming tubes 220 are sufficiently long to ensure that the needles 182 are always completely surrounded by the priming fluid. The back-tracking of fluid itself may be prevented by providing the supply lines 14, needles 182, any conduits interconnecting the supply lines 14 and needles 182 in the connector device 130, and/or the priming tubes 220 with one-way valves which enable travel of the priming fluid in the priming direction only.

In further embodiments, the plurality of priming tubes 220 of the priming device may be replaced by a plurality of indicator conduits, each of which includes a fluid indicator. The fluid indicator may comprise an electronic liquid sensor coupled to an LED or other output device, the LED being arranged to emit light when the liquid sensor detects the presence of liquid within the indicator conduit. The liquid sensor may, for example, comprise a pair of electrodes and means for measuring a drop in electrical resistance between the electrodes when the electrodes are bridged by a liquid. The LED may be replaced by an audible buzzer to alert the clinician to the presence of liquid within the indicator conduit. Alternatively, the electronic liquid sensor may send a signal, either wirelessly or via a wired connection, to a user device having a user interface which indicates the status of the priming operation based on the signal sent by the sensor.

In other embodiments the fluid indicator may comprise a membrane or other substrate which undergoes a visually observable change on contact with liquid. An example of a suitable substrate is litmus paper, which changes colour in response to contact with a liquid.

In such embodiments the plurality of indicator conduits may each include a hydrophilic or hydrophobic membrane to permit venting of air but prevent priming liquid from being expelled from the indicator conduits.

In yet further embodiments, the plurality of priming tubes 220 or plurality of indicator conduits, as appropriate, may each include a stop sensor towards a distal end of the tube or conduit which is arranged to detect the presence of liquid and, if liquid is detected, provide a feedback signal to the pump 18 to tell it to stop delivering priming fluid to the connector device 130.

As mentioned above, the priming device and method according to the present invention may also be used with percutaneous or subcutaneous fluid access apparatus other than the device described above, including such devices which do not include needles. The connector devices of such needleless apparatuses may, for example, have conduits within each of which a normally closed valve can open automatically on connection of the connector device with either the priming device 200 or the percutaneous or subcutaneous fluid access apparatus 100 itself. The conduits may each comprise a micro-tube, for example.

In other embodiments the normally closed valve may not open automatically, but may instead require a user input to achieve opening. Such a valve may comprise a flexible sealing membrane incorporated into each of the fluid conduits, which can open or close to permit or prevent the passage of liquid through the conduits. Alternatively, the valve may comprise a simple open/close valve actuated rotation of a blocking plate which has openings corresponding to each of the conduits, and which can be rotated between an open configuration in which the openings are aligned with the conduits and a closed configuration in which he openings are not aligned with the conduits.

In such needleless embodiments a seal between the conduits of the connector device and the fluid channels of the priming device may be achieved by means of a sealing member such as a gasket. Such a sealing member preferably provides a fluid seal that prevents leakage of fluid or ingress of unwanted material (e.g. bacteria etc.).

Although the invention has been described above with reference to one or more preferred embodiments, it will be appreciated that various changes or modifications may be made without departing from the scope of the invention as defined in the appended claims.

## Claims

1. A system for priming a multi-conduit fluid connector device of a percutaneous fluid access apparatus or subcutaneous fluid access apparatus, comprising:
a multi-conduit fluid connector device comprising a plurality of fluid conduits arranged to receive a priming liquid; and
a priming device comprising multiple fluid channels each for connection to a respective conduit of the multi-conduit fluid connector device to permit delivery of priming liquid from each conduit to a respective fluid channel, and an indicator associated with each respective fluid channel for indicating the presence of priming liquid within the fluid channel.

2. A system according to claim 1, wherein the indicator is a visual indicator to provide a visual indication of the presence of priming fluid within the fluid channel.

3. A system according to claim 2, wherein the visual indicator comprises a length of substantially transparent hollow tube defining the respective fluid channel.

4. A system according to any of claims 1 to 3, wherein the multi-conduit fluid connector device comprises an attachment member configured for attachment to a percutaneous fluid access device and for attachment to the priming device.

5. A system according to claim 4, wherein the attachment member is arranged to orientate the priming device relative to the multi-conduit fluid connector device to ensure alignment between each conduit and its respective fluid channel.

6. A system according to any of claims 1 to 5, wherein the fluid conduits of the multi-conduit fluid connector device each comprise a hollow needle.

7. A system according to claim 6, wherein the multi-conduit fluid connector device has a connection configuration in which the needles do not protrude from the multi-conduit connector device and a connected configuration in which the needles protrude into the fluid channels of the priming device, and the priming device can be connected to or disconnected from the multi-conduit connector device only in the connection configuration.

8. A system according to claim 1, wherein the indicator includes an electronic liquid sensor coupled to an output device.

9. A system according to claim 1, wherein the indicator includes a substrate which is adapted to undergo a visually observable change on contact with liquid.

10. A system according to any preceding claim, comprising a liquid source configured to supply priming fluid through the conduits and into the fluid channels to expel air from the conduits, and optionally from at least a portion of the fluid channels.

11. A system according to any preceding claim, wherein the multi-conduit fluid connector device is configured for attachment to a percutaneous fluid access device or subcutaneous fluid access device comprising an extracorporeal portion, and a plurality of ports accessible from the extracorporeal portion.

12. A method of priming a multi-conduit fluid connector device of a percutaneous fluid access apparatus or subcutaneous fluid access apparatus, the multi-conduit fluid connector device comprising a plurality of fluid conduits, the method comprising:
connecting the multi-conduit fluid connector device to a liquid source;
providing a priming device connected to the multi-conduit fluid connector device, the priming device comprising multiple fluid channels such that each fluid channel is connected to a respective conduit of the multi-conduit fluid connector device, and having an indicator associated with each respective fluid channel,
supplying priming liquid from the liquid source through the conduits and into the fluid channels to expel air from the conduits until each indicator indicates the presence of priming liquid within each of the fluid channels.

13. A method according to claim 12, wherein the fluid conduits of the multi-conduit fluid connector device each comprise a hollow needle, the method further comprising providing the multi-conduit fluid connector device in a connecting configuration in which the hollow needles do not protrude from the multi-conduit fluid connector device, and, only after the multi-conduit fluid connector device is connected to the priming device, deploying the hollow needles to a connected configuration in which they protrude into the fluid channels of the priming device.

14. A method according to claim 13 or claim 14, further comprising, after the presence of priming liquid within each of the channels has been indicated:
disconnecting the priming device from the multi-conduit fluid connector device,
attaching the multi-conduit fluid connector device to a percutaneous fluid access device or subcutaneous fluid access device comprising an extracorporeal portion and a plurality of ports accessible from the extracorporeal portion, such that the conduits provide fluid communication between the conduits and respective ones of the ports.

15. A method according to claim 14, wherein the step of connecting the multi-conduit fluid connector device to the priming device includes connecting the priming device to an attachment member of the multi-conduit fluid connector device, and the step of attaching the multi-conduit fluid connector device to a percutaneous fluid access device includes connecting the percutaneous fluid access device to the attachment member.
